Europäisches Patentamt

European Patent Office

Office européen des brevets

(19)

(11) Veröffentlichungsnummer : **0 531 865 B1**

(12)

# EUROPÄISCHE PATENTSCHRIFT

(45) Veröffentlichungstag der Patentschrift :
**27.04.94 Patentblatt 94/17**

(51) Int. Cl.⁵ : **C07C 303/44,** C07C 309/47

(21) Anmeldenummer : **92114911.8**

(22) Anmeldetag : **01.09.92**

(54) **Verfahren zur Isolierung des Mononatriumsalzes der 2-Naphthylamin-1,5-disulfonsäure.**

(30) Priorität : **12.09.91 DE 4130332**

(43) Veröffentlichungstag der Anmeldung :
**17.03.93 Patentblatt 93/11**

(45) Bekanntmachung des Hinweises auf die
Patenterteilung :
**27.04.94 Patentblatt 94/17**

(84) Benannte Vertragsstaaten :
**CH DE GB IT LI**

(56) Entgegenhaltungen :
**EP-A- 0 037 511
EP-A- 0 074 024**

(56) Entgegenhaltungen :
**EP-A- 0 138 755
F. MUTH '"Methoden zur Herstellung und Umwandlung aromatischer Sulfonsäuren" in ME-
THODEN DER ORGANISCHEN CHEMIE
(HOUBEN-WEYL), 4. Aufl., Band IX' 1955 ,
GEORG THIEME VERLAG , STUTTGART**

(73) Patentinhaber : **BAYER AG
D-51368 Leverkusen (DE)**

(72) Erfinder : **Heidenreich, Holger, Dr.
Blankenese 18
W-2224 Kuden (DE)**
Erfinder : **Behre, Horst, Dr.
Zur alten Linde 12
W-5068 Odenthal-Eikamp (DE)**

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 531 865 B1

**Beschreibung**

Die Erfindung betrifft die Isolierung des Mononatriumsalzes der 2-Naphthylamin-1.5-disulfonsäure (nachfolgend kurz: "2-Naphthylamin-1.5-disulfonsäure-Na") aus diese Säure enthaltenden Sulfiergemischen in verbesserter Qualität.

2-Naphthylamin-1.5-disulfonsäure ("Sulfotobiassäure") und ihr Mononatriumsalz sind wichtige Vorprodukte für die Herstellung von Farbstoffen, insbesondere von Reaktivfarbstoffen. 2-Naphthylamin-1.5-disulfonsäure kann aus 2-Naphthylamin oder 2-Naphthylamin-1-sulfonsäure ("Tobiassäure") durch Sulfierung in rauchender Schwefelsäure ("Oleum") mit einem $SO_3$-Gehalt von 15 bis 30 Gew.-% hergestellt werden.

Aus den entstehenden Reaktionsgemischen kann man 2-Naphthylamin-1.5-disulfonsäure-Na durch Verdünnen mit Wasser in Gegenwart von Natriumionen und Abkühlen der wäßrig-schwefelsauren Lösungen auf Temperaturen um 20°C ausfällen (s. Bucherer u. Wahl, J.pr. Chemie 103, 129 ff. (1921); "Methoden der Organischen Chemie" (Houben-Weyl), Bd. 9, Georg Thieme Verlag, Stuttgart 1955, S. 442, 481-484; Horyna, Jaroslav et. al., Czech. 129866 (1968); Beilstein Bd. XIV H 786, EI 739, EII 475).

Diese Ausfällung des Mononatriumsalzes ist mit dem Nachteil verbunden, daß dabei stark salzhaltige Dünnsäure anfällt, deren Aufarbeitung große technische Schwierigkeiten bereitet. Außerdem enthält das so gewonnene Mononatriumsalz meist größere Mengen (bis zu 15 Gew.-%) Natriumsulfat bzw. Natriumchlorid und anhaftende Restsäure (bis zu 20 Gew.-%), die, da diese Verunreinigungen bei der Weiterverarbeitung stören, durch Waschen mit verdünnter Schwefelsäure und Wasser entfernt werden müssen. Dieses Waschen ist aber mit erheblichen Ausbeuteverlusten verbunden.

Es wurde nun gefunden, daß man die bei der Ausfällung von 2-Naphthylamin-1.5-disulfonsäure-Na auftretenden Schwierigkeiten dadurch vermeiden kann, daß man die Ausfällung durch Verdünnen der Reaktionsgemische unter exakt definierten Bedingungen vornimmt.

Gegenstand der Erfindung ist ein Verfahren zur Isolierung von 2-Naphthylamin-1.5-disulfonsäure-Mononatriumsalz aus einem Reaktionsgemisch, das, bezogen auf das gesamte Reaktionsgemisch, 15 bis 35, vorzugsweise 20 bis 30, Gew.-% 2-Naphthylamin-1.5-disulfonsäure und 65 bis 85, vorzugsweise 70 bis 80, Gew.-% Schwefelsäure oder Oleum mit einem $SO_3$-Gehalt, bezogen auf Schwefelsäure + $SO_3$, von 1 bis 20, vorzugsweise 10 bis 17, Gew.-% enthält, durch

(a) Verdünnen mit verdünnter Schwefelsäure und Wasser auf einen Schwefelsäuregehalt, bezogen auf Schwefelsäure und Wasser, von 20 bis 80, vorzugsweise 35 bis 55, besonders bevorzugt 40 bis 50 und insbesondere 45 bis 48, Gew.-% und Zugabe von Natriumionen abgebenden Verbindungen unter

(b) Aufrechterhaltung einer Temperatur von 30 bis 80°C, vorzugsweise von 35 bis 70°C, besonders bevorzugt von 45 bis 65°C und insbesondere von 54 bis 59°C in einer Weise, daß (i) das Verhältnis von zugesetzter zu ursprünglich vorhandener Schwefelsäure - berechnet als 100 %ige Schwefelsäure - mindestens 0,3, vorzugsweise mindestens 0,5, insbesondere mindestens 0,75, beträgt und (ii) der Schwefelsäuregehalt während des gesamten Verdünnungsvorgangs - ideale Vermischung vorausgesetzt - die Untergrenze des Bereichs (a) um nicht mehr als 10 % unterschreitet.

Wesentlich für das Erzielen des erfindungsgemäßen Effekts ist - neben der Temperatur - nicht nur der Schwefelsäuregehalt am Ende des Verfahrens, sondern offenbar auch das Vermeiden von temporär zu niedrigen Schwefelsäurekonzentrationen, wie sie z.B. beim Einlaufenlassen des Reaktionsgemischs in Wasser durchlaufen werden. Erfindungsgemäß arbeitet man also vorzugsweise so, daß - vom Schwefelsäuregehalt des Reaktionsgemischs aus betrachtet - beim Verdünnen möglichst kein Minimum oder nur ein solches Minimum durchschritten wird, das dem Schwefelsäuregehalt-Endwert eng benachbart ist.

Grundsätzlich kann das erfindungsgemäße Verfahren so ausgeführt werden, daß man das zu verdünnende Reaktionsgemisch und Wasser in verdünnte Schwefelsäure einer Konzentration gibt, die wenigstens den anspruchsgemäß geforderten Schwefelsäuremindestgehalt besitzt, und dann die Natriumverbindung zugibt.

Nach einer bevorzugten Ausführungsform werden Reaktionsgemisch, Wasser, verdünnte Schwefelsäure und Natriumionen abgebende Verbindungen gleichzeitig so dosiert, daß sich die gewünschte Konzentration einstellt und während des gesamten Mischungsvorgangs praktisch konstant gehalten wird.

Anstelle frischer verdünnter Schwefelsäure kann zum Verdünnen auch die nach dem Ausfällen der 2-Naphthylamin-1.5-disulfonsäure verbleibende Schwefelsäure ("Muttersäure") eines vorhergegangenen Ansatzes verwendet werden. Solche "Muttersäure" enthält, bezogen auf die Summe von Wasser und Schwefelsäure, im allgemeinen 20 bis 80, vorzugsweise 35 bis 55, besonders bevorzugt 40 bis 50 und insbesondere 45 bis 48 Gew.-% Schwefelsäure und, bezogen auf Schwefelsäure, 0,1 bis 3,0, vorzugsweise 0,5 bis 1,5 Gew.-% 2-Naphthylamin-1,5-disulfonsäure, 0,01 bis 1,0, vorzugsweise 0,05 bis 0,2 Gew.-% 2-Naphthylamin-1,6-disulfonsäure und 0,01 bis 1,0, vorzugsweise 0,05 bis 0,2 Gew.-% 2-Naphthylamin-1,7-disulfonsäure. Es hat sich herausgestellt, daß die Verwendung von Muttersäure anstelle reiner verdünnter Schwefelsäure zu besserer Filtrierbarkeit und zu einem geringeren Schwefelsäuregehalt des isolierten Produkts führt.

2

Die beim Verdünnungsvorgang anfallende Wärme wird durch entsprechende Kühlung abgeführt.

Als Natriumionen abgebende Verbindungen, die zur Bildung des Mononatriumsalzes zugesetzt werden, kommen vorzugsweise anorganische Natriumverbindungen in Frage. Sie umfassen beispielsweise Natriumchlorid, Natriumsulfat, Natriumhydroxid und Natriumsalze der Kohlensäure wie Natriumcarbonat und Natriumhydrogencarbonat. Sie werden in einer Menge eingebracht, die einem Molverhältnis Natriumverbindung/2-Naphthylamin-1.5-disulfonsäure von mindestens 1, vorzugsweise mindestens 1,5, insbesondere mindestens 2, beträgt. Natriumhydroxid ist die bevorzugteste Natriumverbindung; sie kann am bequemsten in Form von Natronlauge eingesetzt werden. Natronlauge wird vorzugsweise in Form einer 25 bis 50, insbesondere 35 bis 50, speziell 45 bis 50, gew.-%iger Konzentration eingesetzt.

Bei dem erfindungsgemäßen Verfahren fällt das 2-Naphthylamin-1.5-disulfonsäure-Na in einer gut filtrierbaren Form an; der Gehalt an 2-Naphthylamin-1.5-disulfonsäure-Na liegt im Bereich oberhalb von 65 Gew.-%, bezogen auf feuchtes Produkt (oberhalb von 80 Gew.-%, umgerechnet auf trockenes Produkt), und der Gehalt an Schwefelsäure, bezogen auf feuchtes Produkt, liegt unter 1 Gew.-% (berechnet als 100 %ige $H_2SO_4$ und bezogen auf trockenes Produkt).

Es hat sich als vorteilhaft erwiesen, das ausgefällte 2-Naphthylamin-1.5-disulfonsäure-Na noch 0,1 bis 10, vorzugsweise 0,2 bis 5, insbesondere 0,5 bis 2, Stunden bei Temperaturen von 20 bis 55°C, vorzugsweise bei 30 bis 45°C, in der verdünnten Lösung zu belassen, bevor man das Produkt abtrennt.

Das erfindungsgemäße Verfahren kann diskontinuierlich und kontinuierlich ausgeführt werden.

Die Prozentangaben in den nachfolgenden Beispielen beziehen sich auf das Gewicht.

Beispiele

Beispiel 1

In einem 2-1-Fünfhalskolben mit Säbelrührer, 2 Dosiertropftrichtern, Kühler und Innenthermometer werden 600 g 50 %ige Schwefelsäure vorgelegt und auf 40°C erwärmt.

Im Verlaufe von ca. 4 Stunden läßt man bei 40°C simultan unter Kühlung 500 g Sulfiergemisch, das durch Umsetzung von 2-Naphthylamin-1-sulfonsäure (Tobias-Säure) mit Oleum (25 % $SO_3$) bei 20°C erhalten wurde (Gehalt 23,4 % 2-Naphthylamin-1,5-disulfonsäure; 1,5 % 2-Naphthylamin-1,6-disulfonsäure; 0,3 % 2-Naphthylamin-1,7-disulfonsäure; Rest zu 100 % Schwefelsäure, Schwefeltrioxid, organische Nebenprodukte unbekannter Struktur), und 500 g Wasser einlaufen. In die resultierende Kristall-Suspension gibt man anschließend bei 40°C 66 g 50 %ige wäßrige Natronlauge. Es wird 1 Stunde bei 40°C nachgerührt, unter weiterem Rühren im Verlaufe mehrerer Stunden auf 20°C abgekühlt und mehrere Stunden bei 20°C nachgerührt. Das sehr gut filtrierbare Produkt wird auf einer Glassinternutsche abgesaugt und zur Verdrängung der anhaftenden Muttersäure 2 mal mit 225 g Wasser gewaschen. Es werden erhalten (Mittelwert aus 4 Versuchen mit 2 Waschwasserrückführungen):

191 g      2-Naphthylamin-1.5-disulfonsäure-mono-Na-Salz feucht,

1 560 g    Ablauf und

500 g      Waschwasser.

Der durch HPLC bestimmte Gehalt der einzelnen Produkte beträgt durchschnittlich:

EP 0 531 865 B1

> 61,3 % 2-Naphthylamin-1,5-disulfonsäure MG 303
> (freie Säure)
>
> $\hat{=}$ 65,8 % 2-Naphthylamin-1.5-disulfonsäure-Na MG 325
>
> 2,0 % 2-Naphthylamin-1.6-disulfonsäure MG 303
> (freie Säure)
>
> <0,05 % 2-Naphthylamin-1-sulfonsäure MG 223
> (freie Säure)
>
> <0,05 % 2-Naphthylamin-5-sulfonsäure MG 223
> (freie Säure)
>
> <0,05 % 2-Naphthylamin-1.7-disulfonsäure MG 303
> (freie Säure)
>
> 0,4 % Schwefelsäure
>
> 31,6 % Wasser.

Die Ausbeute beträgt 95 % der Theorie, bezogen auf eingesetzte 2-Naphthylamin-1.5-disulfonsäure.

Beispiel 2

293 g Tobiassäure (99,5 % Gehalt) werden mit 1 240 g Oleum (25 % $SO_3$) unter sofortiger Abführung der Reaktionswärme bei einer Temperatur von 18 bis 20°C sulfiert. Nach einer Verweilzeit von 1 Stunde bei 20°C hat das Reaktionsgemisch folgende Zusammensetzung:

> 2-Naphthylamin-1.5-disulfonsäure 24,7 %
> 2-Naphthylamin-1.6-disulfonsäure 0,8 %
> 2-Naphthylamin-1.7-disulfonsäure 0,3 %
> 2-Naphthylamin-1-sulfonsäure 0,1 %
> Schwefelsäure 61,0 %
> $SO_3$ 12,2 %
> $\Sigma$ 100,1 %

1 533 g dieses Reaktionsgemisches werden simultan und kontinuierlich mit 1 550 g Wasser verdünnt und darüber hinaus mit 1 840 g 50 %iger Schwefelsäure und 200 g 50 %iger wäßriger Natronlauge bei 40°C versetzt. Nach 24 Stunden bei 40°C wird filtriert und der Filterkuchen 2 mal mit 480 g Wasser gewaschen. Es werden 568 g eines farblosen Filterkuchens der folgenden Zusammensetzung erhalten:

4

```
    58,6  % 2-Naphthylamin-1,5-disulfonsäure MG 303
             (freie Säure)
≙   62,9  % 2-Naphthylamin-1.5-disulfonsäure-Na
     1,7  % 2-Naphthylamin-1.6-disulfonsäure
     0,05 % 2-Naphthylamin-1.7-disulfonsäure
     0,1  % 2-Naphthylamin-1-sulfonsäure
    33,5  % Wasser
     0,2  % Schwefelsäure
     0,8  % Natriumsulfat
```

Beispiel 3

1 533 g des in Beispiel 2 verwendeten Reaktionsgemisches werden kontinuierlich und simultan mit 1 750 g Waschwasser und 1 840 g rückgeführter Muttersäure aus Beispiel 2 bei 58°C verdünnt. Nach einer Verweilzeit von 1 Stunde bei 58°C wird die partiell ausgefällte Suspension in einen weiteren Rührbehälter abgelassen und dort unter Kühlung mit 150 g 50 %iger wäßriger Natronlauge bei 40°C versetzt. Nach einer weiteren Rührzeit von 23 Stunden wird die Suspension filtriert und der Filterkuchen 2 mal mit 550 g Wasser gewaschen. Man erhält 577 g eines körnigen Filterkuchens folgender Zusammensetzung:

```
    61,6  % 2-Naphthylamin-1,5-disulfonsäure MG 303
             (freie Säure)
≙   66,1  % 2-Naphthylamin-1.5-disulfonsäure-Na
     0,8  % 2-Naphthylamin-1.6-disulfonsäure
    <0,05 % 2-Naphthylamin-1.7-disulfonsäure
    <0,05 % 2-Naphthylamin-1-sulfonsäure
    33,1  % Wasser
     0,3  % Schwefelsäure
```

Vergleich

1 533 g des in Beispiel 2 verwendeten Reaktionsgemisches werden auf ein Gemisch von 730 g Wasser und 2 500 g Eis so abgedrückt, daß die Temperatur von 20°C nicht überschritten wird. Danach werden 500 g Kochsalz eingetragen, mehrere Stunden nachgerührt und filtriert. Der Filterkuchen wird mit 10 %iger wäßriger Kochsalzlösung "säurefrei" gewaschen. Man erhält 652 g einer Feuchtpaste folgender Zusammensetzung:

```
55,0  %  2-Naphthylamin-1,5-disulfonsäure MG 303
         (freie Säure)
≙  59,0  %  2-Naphthylamin-1.5-disulfonsäure-Na
    2,2  %  2-Naphthylamin-1.6-disulfonsäure
   0,05 %  2-Naphthylamin-1.7-disulfonsäure
   0,05 %  2-Naphthylamin-1-sulfonsäure
   37,8  %  Wasser
    0,2  %  Schwefelsäure
    0,6  %  Natriumchlorid
```

Beispiel 4

1 533 g des in Beispiel 2 verwendeten Reaktionsgemisches werden kontinuierlich und simultan mit 1 800 g Waschwasser, 1 840 g rückgeführter Muttersäure aus Beispiel 2 und 200 g 50 %iger wäßriger Natronlauge bei 55°C verdünnt. Nach einer Verweilzeit von 1 Stunde bei 55°C wird die partiell ausgefällte Suspension in einen weiteren Rührbehälter abgelassen und dort auf 40°C gekühlt. Nach einer weiteren Rührzeit von 23 Stunden wird die Suspension Filtriert und der Filterkuchen dreimal mit je 400 g Wasser gewaschen. Man erhält 490 g eine körnigen Filterkuchens folgender Zusammensetzung:

```
77,6  %  2-Naphthylamin-1,5-disulfonsäure MG 303
         (freie Säure)
≙  83,2  %  2-Naphthylamin-1.5-disulfonsäure-Na
    1,3  %  2-Naphthylamin-1.6-disulfonsäure
    0,1  %  2-Naphthylamin-1.7-disulfonsäure
   0,06 %  2-Naphthylamin-1-sulfonsäure
   15.5  %  Wasser
    0,6  %  Schwefelsäure
```

**Patentansprüche**

1. Verfahren zur Isolierung von 2-Naphthylamin-1.5-disulfonsäure-Mononatriumsalz aus einem Reaktionsgemisch, das, bezogen auf das gesamte Reaktionsgemisch, 15 bis 35 Gew.-% 2-Naphthylamin-1.5-disulfonsäure und 65 bis 85 Gew.-% Schwefelsäure oder Oleum mit einem $SO_3$-Gehalt, bezogen auf Schwefelsäure + $SO_3$, von 1 bis 20 Gew.-% enthält, durch
   (a) Verdünnen mit verdünnter Schwefelsäure und Wasser auf einen Schwefelsäuregehalt, bezogen auf Schwefelsäure und Wasser, von 20 bis 80 Gew.-% und Zugabe von Natriumionen abgebenden Verbindungen unter
   (b) Aufrechterhaltung einer Temperatur von 30 bis 80°C in einer Weise, daß (i) das Verhältnis von zugesetzter zu ursprünglich vorhandener Schwefelsäure - berechnet als 100 %ige Schwefelsäure - mindestens 0,3 beträgt und (ii) der Schwefelsäuregehalt während des gesamten Verdünnungsvorgangs - ideale Vermischung vorausgesetzt - die Untergrenze des Bereichs (a) um nicht mehr als 10 % unterschreitet.

2. Verfahren nach Anspruch 1, wonach ein Reaktionsgemisch mit einem Gehalt von 20 bis 30 Gew.-% 2-Naphthylamin-1.5-disulfonsäure eingesetzt wird.

**3.** Verfahren nach Anspruch 1, wonach ein Reaktionsgemisch mit einem Gehalt von 70 bis 80 Gew.-% Oleum eingesetzt wird.

**4.** Verfahren nach Anspruch 1, wonach das Oleum einem SO$_3$-Gehalt von 10 bis 17 Gew.-% besitzt.

**5.** Verfahren nach Anspruch 1, wonach man auf einen Schwefelsäuregehalt von 35 bis 55 Gew.-% verdünnt.

**6.** Verfahren nach Anspruch 1, wonach man auf einen Schwefelsäuregehalt von 40 bis 50 Gew.-% verdünnt.

**7.** Verfahren nach Anspruch 1, wonach man auf einen Schwefelsäuregehalt von 45 bis 48 Gew.-% verdünnt

**8.** Verfahren nach Anspruch 1, wonach man in einem Temperaturbereich von 35 bis 70°C arbeitet.

**9.** Verfahren nach Anspruch 1, wonach das Verhältnis (i) mindestens 0,5 beträgt.

**10.** Verfahren nach Anspruch 1, wonach man gleichzeitig mit Wasser und verdünnter Schwefelsäure verdünnt.

**11.** Verfahren nach Anspruch 1, wonach man anstelle von verdünnter Schwefelsäure Muttersäure eines vorhergegangenen Ansatzes verwendet.

## Claims

**1.** Process for the isolation of 2-naphthylamine-1,5-disulphonic acid monosodium salt from a reaction mixture which comprises, based on the total reaction mixture, 15 to 35% by weight of 2-naphthylamine-1,5-disulphonic acid and 65 to 85% by weight of sulphuric acid or oleum having an SO$_3$ content, based on the sulphuric acid + SO$_3$, of 1 to 20% by weight, by
(a) dilution with dilute sulphuric acid and water to a sulphuric acid content, based on the sulphuric acid and water, of 20 to 80% by weight and addition of compounds which donate sodium ions while
(b) maintaining a temperature of 30 to 80°C in a manner such that (i) the ratio of sulphuric acid added to sulphuric acid originally present - calculated as 100% strength sulphuric acid - is at least 0.3 and (ii) the sulphuric acid content throughout the entire dilution operation - provided mixing is ideal - falls below the lower limit of range (a) by not more than 10%.

**2.** Process according to Claim 1, in which a reaction mixture containing 20 to 30% by weight of 2-naphthylamine-1,5-disulphonic acid is employed.

**3.** Process according to Claim 1, in which a reaction mixture containing 70 to 80% by weight of oleum is employed.

**4.** Process according to Claim 1, in which the oleum has an SO$_3$ content of 10 to 17% by weight.

**5.** Process according to Claim 1, in which the mixture is diluted to a sulphuric acid content of 35 to 55% by weight.

**6.** Process according to Claim 1, in which the mixture is diluted to a sulphuric acid content of 40 to 50% by weight.

**7.** Process according to Claim 1, in which the mixture is diluted to a sulphuric acid content of 45 to 48% by weight.

**8.** Process according to Claim 1, which is carried out in a temperature range of 35 to 70°C.

**9.** Process according to Claim 1, in which the ratio (i) is at least 0.5.

**10.** Process according to Claim 1, in which the mixture is diluted simultaneously with water and dilute sulphuric acid.

**11.** Process according to Claim 1, in which the mother acid of a previous batch is used instead of dilute sulphuric acid.

7

**Revendications**

1. Procédé d'isolation de sel monosodique d'acide 2-naphtylamine-1,5-disulfonique à partir d'un mélange réactionnel qui contient, par rapport au mélange réactionnel total, 15 à 35 % en poids d'acide 2-naphtylamine-1,5-disulfonique et 65 à 85 % en poids d'acide sulfurique ou d'oléum avec une teneur en $SO_3$, par rapport à l'acide sulfurique + le $SO_3$, comprise entre 1 et 20 % en poids, par

   (a) dilution avec de l'acide sulfurique dilué et de l'eau à une teneur en acide sulfurique, par rapport à l'acide sulfurique et l'eau, comprise entre 20 et 80 % en poids et addition de composés donneurs d'ions sodium, avec

   (b) maintien d'une température comprise entre 30 et 80°C d'une façon telle que (i) le rapport de l'acide sulfurique ajouté à l'acide sulfurique d'origine - compté sous forme d'acide sulfurique à 100 % - s'élève à au moins 0,3 et que (ii) la teneur en acide sulfurique pendant tout le déroulement de la dilution - on suppose le mélange idéal - ne descend pas en dessous de plus 10 % de la limite inférieure de l'intervalle (a).

2. Procédé selon la revendication 1, dans lequel on utilise un mélange réactionnel ayant une teneur en acide 2-naphtylamine-1,5-disulfonique comprise entre 20 et 30 % en poids.

3. Procédé selon la revendication 1, dans lequel on utilise un mélange réactionnel ayant une teneur en oléum comprise entre 70 et 80 % en poids.

4. Procédé selon la revendication 1, dans lequel l'oléum présente une teneur en $SO_3$ comprise entre 10 et 17 % en poids.

5. Procédé selon la revendication 1, dans lequel on dilue à une teneur en acide sulfurique de 35 à 55 % en poids.

6. Procédé selon la revendication 1, dans lequel on dilue à une teneur en acide sulfurique de 40 à 50 % en poids.

7. Procédé selon la revendication 1, dans lequel on dilue à une teneur en acide sulfurique comprise entre 45 et 48 % en poids.

8. Procédé selon la revendication 1, dans lequel on travaille dans un intervalle de température de 35 à 70°C.

9. Procédé selon la revendication 1, dans lequel le rapport (i) vaut au moins 0,5.

10. Procédé selon la revendication 1, dans lequel on dilue en même temps avec de l'eau et de l'acide sulfurique dilué.

11. Procédé selon la revendication 1, dans lequel on utilise, à la place de l'acide sulfurique dilué, l'acide mère d'une masse réactionnelle antérieure.